# EUROPEAN PATENT SPECIFICATION

(11) **EP 2 558 123 B1**
(45) Date of publication and mention of the grant of the patent: **09.08.2023**
(21) Application number: 11769425.7
(22) Date of filing: 12.04.2011
(51) Int. Cl.: A61K 39/39, A61K 35/12, A61P 31/00, A61P 31/04, A61P 37/02, A61P 37/04, A61P 43/00, A61K 38/17, A61K 38/21, A61K 39/00, A61K 35/17, C12N 5/0783, A61P 35/00

(54) **USES AND COMPOSITIONS FOR INHIBITION OF TREG CELLS, COMPRISING T HELPER CELLS AND CHAPERONINE-RICH CELL LYSATE**
VERWENDUNGEN UND ZUSAMMENSETZUNG ZUR HEMMUNG VON TREG-ZELLEN, ENTHALTEND T-HELPER ZELLEN UND EIN ZELLLYSAT ANGEREICHERT FÜR CHAPERONINEN
UTILISATIONS ET COMPOSITIONS POUR INHIBER LES LYMPHOCYTES TREG, COMPRENANT DES CELLULES T HELPER ET UN LYSTA CELLULAIRE RICHE EN CHAPERONINES

(30) Priority: 13.04.2010 US 323557 P
(43) Date of publication of application: 20.02.2013
(73) Proprietor: Immunovative Therapies, Ltd., 60850 Shoham (IL)
(72) Inventor: HAR-NOY, Michael, 71700 Modiin (IL)
(74) Representative: Wilson, Justin Scott
(86) International application number: PCT/US2011/032090
(87) International publication number: WO 2011/130249

(56) References cited:
- WO-A2-2009/135199
- WO-A2-2011/084451
- US-A1- 2008 112 963
- US-A1- 2010 086 561
- M. HAR-NOY ET AL.: 'Allogeneic CD3/CD28 cross-linked Th1 memory cells provide potent adjuvant effects for active immunotherapy of leukemia/ lymphoma.' LEUKEMIA RESEARCH vol. 33, no. 4, 2009, ISSN 0145-2126 pages 525 - 538
- MICHAEL W. GRANER ET AL.: 'Tumor-derived chaperone-rich cell lysates are effective therapeutic vaccines against a variety of cancers.' CANCER IMMUNOL. IMMUNOTHER. vol. 52, no. 4, 2003, ISSN 0340-7004 pages 226 - 234
- NICOLAS LARMONIER ET AL.: 'Chaperone-rich tumor cell lysate-mediated activation of antigen-presenting cells resists regulatory T cell suppression.' JOURNAL OF LEUKOCYTE BIOLOGY vol. 83, no. 4, 2008, ISSN 0741-5400 pages 1049 - 1059
- DATABASE BIOSIS [Online] BIOSCIENCES INFORMATION SERVICE, PHILADELPHIA, PA, US; January 2009 (2009-01), RUTER JENS ET AL: "Altering regulatory T cell function in cancer immunotherapy: a novel means to boost the efficacy of cancer vaccines", Database accession no. PREV200900149710 & FRONTIERS IN BIOSCIENCE, vol. 14, January 2009 (2009-01), pages 1761-1770, ISSN: 1093-9946, DOI: 10.2741/3338
- DATABASE BIOSIS [Online] BIOSCIENCES INFORMATION SERVICE, PHILADELPHIA, PA, US; May 2007 (2007-05), CURIEL TYLER J: "Tregs and rethinking cancer immunotherapy", Database accession no. PREV200700387592 & JOURNAL OF CLINICAL INVESTIGATION, vol. 117, no. 5, May 2007 (2007-05), pages 1167-1174, ISSN: 0021-9738, DOI: 10.1172/JCI31202
- HAR-NOY M ET AL: "Allogeneic CD3/CD28 cross-linked Th1 memory cells provide potent adjuvant effects for active immunotherapy of leukemia/lymphoma", LEUKEMIA RESEARCH, NEW YORK,NY, US, vol. 33, no. 4, 1 April 2009 (2009-04-01), pages 525-538, XP025951806, ISSN: 0145-2126, DOI: 10.1016/J.LEUKRES.2008.08.017 [retrieved on 2008-10-01]
- N. Janikashvili ET AL: "Allogeneic effector/memory Th-1 cells impair FoxP3+ regulatory T lymphocytes and synergize with chaperone-rich cell lysate vaccine to treat leukemia", Blood, vol. 117, no. 5, 3 February 2011 (2011-02-03), pages 1555-1564, XP055034790, ISSN: 0006-4971, DOI: 10.1182/blood-2010-06-288621

## Description

### BACKGROUND

The primary objective of cancer immunotherapy is to promote tumor elimination through the activation of innate and adaptive immune responses and relies on the development of vaccination strategies endowed with the dual capability of inducing tumor-specific lymphocytes while overcoming the mechanisms of immune tolerance. Use of cancer immunotherapy can potentially result in tumor elimination and durable remissions with properly designed cancer vaccines. The specificity in immunotherapy of targeting malignant cells and avoiding normal cells can also lead to minimal toxicity.

Immune responses are generally described by two polarized responses, a Th1 (T-helper) response and a Th2 response. A Th1 response mediates cellular immunity and is critical for immune-mediated tumor eradication and a Th2 response mediates humoral immunity. A Th2 response can protect against some infectious diseases but is not desired for an anti-tumor response. Th1 and Th2 responses are counter-regulatory, increased Th1 responses down regulate Th2 response and vice versa.

While Th1 immunity is essential for tumor eradication, in some patients the tumor can continue to grow in the presence of a Th1 environment. Tumors, therefore, can successfully suppress and avoid a protective Th1 response. A successful immunotherapy has to promote a Th1 environment and also disable tumor avoidance mechanisms and counter natural tolerance circuits that prevent immune destruction of normal tissues.

Many of the mechanisms that impede anti-tumor immunity are a result of the activity of CD4+CD25+FoxP3+ T regulatory (Treg) cells. These Treg cells critically contribute to the occurrence and persistence of tumor -induced tolerance and are the dominant immune escape mechanism in early tumor progression. Treg expansion observed during tumor progresssion may result from the proliferation of naturally occurring Tregs (nTregs) or from conversion of CD4⁺CD25⁻FoxP3⁻ T cells into CD4⁺CD25⁺FoxP3⁺ Tregs (iTregs). Tregs dampen immune responses by suppressing the function of the effectors CD4+, CD8+, and natural killer (NK) cells and by inhibiting dendritic cell activation. Because Tregs are one of the main barriers for the eradication of tumors by immune cells, their therapeutic depletion or their functional inactivation using drugs or antibodies improves responses to cancer immunotherapy. However, the selective elimination or inactivation of Tregs constitutes a major challenge because these cells share the same surface markers as activated conventional, nonsuppressive T cells. Antibody-based approaches, for example, indistinguishably target both Tregs and activated effector T lymphocytes.

The use of an anti-cancer vaccine containing CRCL (Chaperone Rich Cell Lysate) has been shown to provide immunostimulatory effects. The CRCL is generated from tumor cell lysates by an isoelectric focusing technique. CRCL-treated dendritic cells and macrophages resist Treg inhibition and retain the production of pro-inflammatory cytokines and immunostimulatory potential. However, a direct effect of CRCL on Treg seems to be unlikely since the survival of Treg, FoxP3 expression and immunosuppressive function is maintained following exposure to CRCL.

Several chemotherapeutic agents (i.e. cyclophosphomide, imatinib mesylate) have been described as the negative modulators of Treg function further improving cancer vaccine efficacy. Adoptive transfer of allogeneic T cells may also increase the efficacy of tumor immunotherapy by providing adjuvant "danger" signals to the host immune cells. This effect partly stems from cytokine production by activated T lymphocytes, which foster the development of protective type-1 immune responses. However, the effects of type I cytokines, including IFN-γ, on Treg have been inconsistent. As an essential effector cytokine for cell-mediated immunity, exogenous or autocrine IFN-γ can negatively regulate Treg generation. In parallel with this concept, other studies have found that IFN-γ enhances activation-induced cell death and that this cytokine might regulate the expansion and persistence of effector T cells by enhancing intrinsic and extrinsic pathway-dependent apoptosis.

### SUMMARY

In one aspect, the present invention relates to a therapeutic composition for use in treating a cancerous tumour in a patient, the composition comprising activated allogeneic emTh-1 cells and a chaperone rich cell lysate derived from the tumour, wherein the composition generates tumour-specific immunity in the patient such that the composition decreases the immunosuppressive activity of tumour-induced Treg cells in the patient while simultaneously promoting a therapeutic effect in the patient, and wherein, in use, the emTh-1 cells and the tumour-derived chaperone rich cell lysate are administered 3-6 times and at intervals of about 3-10 days by intradermal injections at the same location, followed then by an intravenous infusion of the emTh-1 cells.

### BRIEF DESCRIPTION OF THE DRAWINGS

Fig. 1A is a dot plot that shows the effect on FoxP3 expression when naive T cells are resuspended with different tumor cell lines and treated with activated emTh-1 cells
Fig. 1B is a bar graph that shows the effect on FoxP3 expression when naive T cells are resuspended with different tumor cell lines and treated with activated emTh-1 cells.
Fig. 2A is a dot plot that shows the effect on FoxP3 expression when naive T cells are cultured with T-cell expander beads either with or without TGF-β1, in the presence or absence of emTh-1 supernatant.
Fig. 2B is a histogram that shows the effect on FoxP3 expression when naive T cells are cultured with T-cell expander beads either with or without TGF-β1, in the presence or absence of emTh-1 supernatant.
Fig. 2C is a bar graph showing percentage of CD4⁺CD25⁻FoxP3⁻ activated T cells in total CD4⁺ T lymphocytes.
Fig. 2D is a dot plot showing the expression of transcription factors FoxP3, Tbet, and GATA-3 in cells cultured as described in Fig. 2A.
Fig. 2E is a bar graph of the expression of FoxP3 in cells cultured as described in Fig. 2A with the indicated concentration of emTh-1 supernatant.
Fig. 2F is a bar graph of the immunosuppressive activity of residual FoxP3-expressing cells generated in the presence of emTh-1 supernatant.
Fig. 2G is a bar graph that shows emTh-1 supernatant inhibiting the suppressive activity of converted iTreg cells.
Fig. 3A is a bar graph that shows naive T cells with T-cell expander beads with or without TGF-β1 in the presence or absence of emTh-1 supernatant with or without blocking antibodies against mouse IFN-γ.
Fig. 3B is a bar graph that shows naive T cells with T-cell expander beads with or without TGF-β1 in the presence or absence of emTh-1 supernatant with or without blocking antibodies against against mouse TNF-α .
Fig. 3C is a dot plot of the FoxP3 expression in naive T lymphocytes isolated from IFN-γR^{-/-} mouse spleens and cultured as described in Fig. 3A.
Fig. 3D is a bar graph that shows the percentage FoxP3 expressing cells isolated from IFN-γR^{-/-} mouse spleens and cultured as described in Fig. 3A.
Fig. 4A is a plot showing nTreg (CD4⁺CD25⁺Treg) immunosuppressive function inhibited by emTh-1.
Fig. 4B is a bar graph showing nTreg (CD4⁺CD25⁺Treg) immunosuppressive function inhibited by emTh-1.
Fig. 4C is a bar graph nTreg (CD4⁺CD25⁺Treg) immunosuppressive function inhibited by emTh-1.
Fig. 4D is a bar graph showing the IFN-γ concentration in cells described for Fig. 4A.
Fig. 4E is a plot that shows human emTh-1 supernatant reduces human Treg suppressive function and promotes effector T-cell resistance to Treg-mediated inhibition.
Fig. 4F is a plot that shows human emTh-1 supernatant reduces human Treg suppressive function and promotes effector T-cell resistance to Treg-mediated inhibition.
Fig. 5A is a graph showing the effect of vaccines on naive Balb/c mice injected with 12B 1 cells and administered CRCL, emTh-1 or a combination of both CRCL and emTh-1.
Fig. 5B is a graph showing the effect of vaccines on naive Balb/c mice injected with 12B 1 cells and administered CRCL, emTh-1 or a combination of both CRCL and emTh-1.
Fig. 5C is a graph showing the effect of vaccines on naive Balb/c mice injected with 12B 1 cells and administered a combination of both CRCL and emTh-1 or CRCL, emTh-1 and anti-asialo GM1 antibodies
Fig. 5D is graph showing the effect of vaccines on C57BL/6 mice injected with B16 cells and administered a combination of emTh-1 and B 16 CRCL.
Figs. 6A-6D is a plot showing the tumor volume in mice and is indicative of the tumor specific immunity induced by emTh-1 plus CRCL immunotherapy.
Fig. 7A is a bar graph showing the effects of emTh-1 cells plus CRCL immunotherapy on antitumoral T lymphocytes.
Fig. 7B is a dot plot showing that emTh-1 cells skew the differentiation of naive T lymphocytes toward CD4⁺CD25⁺FoxP3⁻ effector T cells rather than Tregs in vivo.
Fig. 7C is a bar graph showing that emTh-1 cells impair Treg suppressive function in vivo.

### DETAILED DESCRIPTION

The present invention includes a method for suppressing immune tolerance of disease antigens and/or disabling tumor immunoavoidance mechanisms in a patient. The method also includes stimulating immunity against the disease antigens, in particular Th1 immunity. Both the suppression of immune tolerance and stimulation of anti-disease antigen immunity is developed by the administration of a therapeutic composition that includes activated allogeneic emTh-1 cells and a chaperone rich cell lysate derived from the tumour.

In the present invention, the method for suppression of immune tolerance includes decreasing the immunosuppressive activity of tumour-induced Treg cells. The method can inhibit the conversion of naive T-cells (CD4+CD25-FoxP3-) to Treg cells (CD4+CD25+FoxP3+), referred to herein as iTreg. The suppressive activity of both naturally occuring Treg (nTreg) cells and iTreg cells can be reduced or eliminated. The methods of the present invention advantageously inhibit the immunosuppressive activity of Treg while simultaneously promoting the function of conventional effector T lymphocytes in the patient. The inhibitory activity of the therapeutic composition on Treg is dependent on IFN-γ, which in preferred compositions is produced by the em-Th1 cells which in turn increase the production of IFN-γ from host innate and adaptive immune cells.

"Suppression of immune tolerance" as referred to herein relates to suppressing the ability of the patient's immune system to tolerate the presence of disease antigens including any natural tolerance and/or suppression of tumor avoidance by the patient's immune system.

The methods described herein can be used to treat a variety of cancerous tumors. The tumors may be present in a variety of locations in the patient including breast, lungs, fiver, stomach, skin, pancreas and the like. The patients can be human or non-human.

The methods of the present invention include administering a therapeutic composition to a patient wherein administration of the composition can suppress the tolerance of disease and/or disease antigens by the patient's immune system. Administration of the therapeutic composition to a patient can disable the tumor avoidance mechanisms in a patient and lead to better tumor eradication. Preferably, administration of the therapeutic composition can suppress the activity of Treg cells that are CD4+CD25+FoxP3+. "Treg" cells as referred to herein relate to regulatory T-cells that are CD4+CD25+FoxP3+ and include both nTreg and iTreg. Naive T-cells as referred to herein are T-cells that are CD4+CD25-FoxP3-.

The methods of the present invention include suppressing the activity of Treg cells in a variety of ways. The suppression of the activity of Treg cells can be, for example, by inhibiting the conversion of naive T-cells to iTreg cells. The method includes administration of therapeutic compositions described herein that interfere with the conversion of naive cells to Treg cells, for example, by modulating the activity of FoxP3. FoxP3 is a transcription factor that is a marker for cells which are capable of causing immune suppression activity. The absence or reversal of FoxP3 in a cell is an indication that the cell does not perform suppressive functions.

The method of the present invention can also negatively modulate a patient's nTreg cells in order to enhance the suppression of immune tolerance. A patient can have nTreg cells in addition to iTreg cells derived from naive T cells in response to a stimulus. The therapeutic composition used in the methods of the present invention may also suppress the activity of nTreg and/or iTreg cells in the patient.

The methods can also include the administration of IFN-gamma in order to suppress the activity of the Treg cells and/or the inclusion of CD40L. The IFN-gamma and/or CD40L can be used as part of the therapeutic composition. The use of IFN-gamma and/or CD40L in the therapeutic composition can block the immunosuppressive activity of Treg without the hindering the activity of the effector T lymphocytes. Preferrably, the IFN-gamma is produced naturally from activated Th1 cells whereby these cells also express CD40L on the surface which can upregulate the expression of IL-12 in host immune cells leading to increased host IFN-gamma production.

The methods of the present invention can also include skewing the differentiation of the naive T-cells to CD4+CD25+Tbet+GATA3- phenotype (Tbet+ cells). Administration of the therapeutic composition can skew the conversion of naive T-cells to the Tbet+ cells which are indicative of a Th1 immune environment in a patient. The presence of Tbet+ can lead to promotion of a Th1 environment and to suppression of the immune tolerance activity due to the promotion away from the production of Treg cells.

The present invention includes administering a therapeutic composition that suppresses the activity of Treg cells as described in the methods above. The therapeutic composition includes living immune cells, where at least a portion are T-cells. The T-cells are effector/memory T-cells (CD45RO+, CD62L^{Lo}) of the Th1 phenotype (CD4+ T-cells that produce IFN-γ and not IL-4). The effector/memory Th1 cells are activated at the time of formulation and introduction to a patient and referred to herein as emTh-1 cells. The preferred activation method is by the cross-linking of CD3 and CD28 surface molecules on the T-cells. Other activation methods are also within the scope of the invention. The emTh-1 cells preferably express CD40L upon being activated and produce large amounts of inflammatory cytokines (such as IFN- γ, GM-CSF, and TNF-α). These emTh-1 cells are preferably allogeneic to the patient. Activation of emTh-1 can be performed as described, for example, in US Patent No. 7,435,592 to Har-Noy and formulated for administration in buffer as described in US Patent No. 7,402,431 to Har-Noy. Activated emTh-1 cells are used in the therapeutic composition described herein. Such activated e-mTh1 cells are available under the trademark ALLOSTIM from Immunovative Therapies, Ltd. of Israel.

In addition to emTh-1 cells, the therapeutic composition includes a chaperone rich cell lysate derived from the tumour that enhances the maintenance of a Th1 environment in the patient and/or suppress the immune tolerance of disease antigens by the patient. In some preferred embodiments, the therapeutic composition can also include antigens associated with the disease. The therapeutic composition can include one or more disease antigens. If more than one antigen is included in the composition, the antigens may be from the same antigen source or different antigen sources. Any antigen source can be used in the formulation, for example these antigens can be sourced from living cells or organisms, the source material can be freeze/thaw lysates, irradiation inactivated (or other inactivation method), used as whole cells or organisms or lysates therefrom. In some preferred embodiments, tumor cells or tumor cell lysates can serve as the cell source material for the antigens. The cell source material can be derived from autologous or allogeneic cell sources or from cell lines. Antigens can also be sourced from naked DNA or RNA, which encode for antigens. The nuclear material can be used alone or incorporated with viral vectors. Another example of antigen source is anti-idiotypic antibodies or portions thereof that mimic antigens, or other methods to mimic the structure of an antigen. Antigen-pulsed or transfected dendritic cells (DC) can also be an antigen source in the composition. The DC can be pulsed with peptides or whole proteins, recombinant proteins, or mRNA or DNA encoding for antigen(s), or the DC can be fused with cells containing the antigens, or the DC can be transfected with viral vectors such as retrovirus, lentivirus, adenovirus which contain the antigen, or these antigen source components can be used alone without the DC.

One or more tumor associated antigens (TAA) can also be used in the therapeutic composition, examples of TAA include: MART-1, gp100, tyrosinase, Melan A, TRP-1, tumor-specific mutated gene products, such as CDK-4, β-catenin, MUM-1, oncogenes such as p53, and ras (K-and H-ras), cancer testes antigens, such as MAGE, GAGE and NY-ESO1, over-expressed self antigens such as MUC1, cyclin B1, Her2-neu, CEA, WT, p53, SART-1, PRAME, p15, and viral antigens such as HPV E7, EBV-derived antigens and telomerase.

In a preferred embodiment, the antigenic component can include one or more chaperone proteins (also known as heat shock proteins) isolated from dead infected tissue or tumors. Heat shock proteins (HSP) are among the major targets of the immune response to bacterial, fungal and parasitic pathogens. Tumor derived heat shock protein (hsp)-peptide complexes (particularly hsp70 and grp94/gp96) have been demonstrated to serve as effective vaccines, producing anti-tumor immune responses in animals and in man. This approach utilizes the peptide binding properties of stress proteins that are responsible for their functions as molecular chaperones in numerous cellular processes.

Certain chaperones in extracellular milieu can also be capable of modulating innate and adaptive immunity due to their ability to chaperone polypeptides and to interact with the host's immune system, particularly professional antigen-presenting cells. Vaccination with HSP from tumors can elicit an anti-tumor response, and down-regulate immune suppression mechanisms. The immunogenicity of HSPs can be derived from the antigenic peptides with which they associate.

A preferred method for isolation of chaperone proteins for use as an antigen source is described by Katsantis in US Pat No. 6,875,849. Additional methods are described by Srivastava in US Pat Nos. 6,797,480; 6,187,312, 6,162,436; 6,139,841; 6,136,315; and 5,837,251. The HSP can also be pulsed with antigens, including peptides, whole cells or cell lysates.

Tumor-derived Chaperone Rich Cell Lysate (CRCL) is used as an antigen source and is obtained by the enrichment of the major chaperone proteins from tumor lysate using a free solution-isolectric focusing (FS-IEF) technique as described in the Examples below. This technique is a rapid and efficient procedure for obtaining up to 5 to 20 times more antigenic material and in less time compared to conventional techniques. The FS-IEF method of multiple chaperone complex enrichment can be desirable from a clinical standpoint in terms of high yield from a potentially limited tumor source, and with a rapid turn-around time from tumor harvest to treatment of the patient.

In addition, the antigens conventionally used in vaccines can also be used in the pharmaceutical composition of the present invention, including whole microorganisms or part(s) of the microorganisms such as live attenuated whole microorganisms, inactivated microorganisms, recombinant peptides and proteins, glycoproteins, glycolipids, lipopeptides, synthetic peptides, or ruptured microorganisms, polysaccharides, used alone or conjugated to carrier elements, such as carrier proteins, can also be used.

In general, any antigen or combination of antigens that are capable of being used for the treatment or prevention of diseases can be used in the therapeutic composition. Antigens derived from infectious pathogens can also serve as antigen sources and may be referred to herein as disease causing antigens. Examples of diseases from which antigens can be sourced are: diphtheria, tetanus, polio, rabies, whooping cough, hepatitis A, B and C, EBV, CMV, herpes 1 and 2, yellow fever, typhoid fever, chicken pox, variola (small pox), measles, mumps, German measles, Japanese encephalitis, meningitis, influenza, pneumococcal infections, rotavirus infections, AIDS (HIV1 and 2), cancers, HTLV1 and 2, syphilis, HPV, tuberculosis, Lyme disease, RSV infections, Trypanosomiasis, Dengue fever, malaria, anthrax, ebola virus, tularemia, Yersinia, West Nile virus, bacterial ailments caused by Chlamydia, Neisseria gonorrheae, Streptococcus pneumoniae, Moraxella catarrhalis, Staphylococcus aureus or Haemophilus influenza type B, malaria, leishmaniasis, listeriosis, etc.

The therapeutic composition includes emTh-1 cells and also the CRCL lysate. The emTh-1 cells and the CRCL lysate individually promotes Th1 immunity but the addition of the emTh-1 cells provides an adjuvant to increase the development of Th1 immunity and also promote suppression of the Treg activity. In addition, a synergistic response can be seen when these two components are combined in the therapeutic composition such that the combination is more effective than either composition alone.

The therapeutic composition may also include other components or factors that are capable of interfering with decreasing or inhibiting the immune tolerance activity of Treg cells. In one embodiment, the therapeutic composition may include exogenous IFN-gamma. The IFN-gamma may be a purified or a recombinant IFN-gamma. The therapeutic composition may also include anti-TGF-beta antibodies. TGF-beta generally increases the Treg cell activity. The inclusion of anti-TGF-beta antibodies in the therapeutic composition can decrease the activity of Treg cells.

The allogeneic emTh-1 cells are preferably derived from a deliberately HLA-mismatched donor. Preferred dosage in a therapeutic composition is at least about 1 × 10⁷ cells for intradermal route and more preferred is between about 5 × 10⁷ to 1 × 10⁹ cells for intravenous route.

The emTh1 cells are first administered intradermally and then a CRCL composition is administered intradermally in the same location. These intradermal injections are repeated about 3-6 times and about 3-10 days apart. In order then to activate memory cells generated by this vaccination schedule, an intravenous infusion of the emTh1 cells alone is administered.

The present disclosure also includes a method for stimulating a therapeutic immune effect in a patient comprising decreasing the immunosuppressive activity of Treg cells by administering a therapeutic composition comprising allogeneic Th1 cells to a patient wherein decreasing and/or inhibiting the activity of the Treg cells induces a therapeutic effect against the disease in the patient. The present disclosure also includes a method of impairing the immunosuppressive functions in a patient comprising decreasing the immunosuppressive activity of Treg cells by administering a therapeutic composition comprising allogeneic Th1 cells to a patient wherein decreasing and/or inhibiting the activity of the Treg cells reduces the immunosuppressive functions in the patient.

### EXAMPLES

### Materials and Methods

Mice-Mice were housed under specific pathogen-free conditions and cared for according to the guidelines of the University of Arizona Institutional Animal Care and Use Committee. Female BALB/c (H2^{d}), C57BL6 (H2^{b}), severe combined immunodeficiency SCID (H2^{d}) and Nude (H2^{d}) mice were obtained from the National Cancer Institute (Bethesda, MD). IFN-γ-Receptor^{-/-} (H2^{d}) mice were purchased from the Jackson Immunoresearch Laboratories (Sacramento, CA). FoxP3-EGFP (H2^{d}) mice that co-express greeen fluorescent protein (GFP) and FoxP3 under the control of the endogenous promoter were obtained from Jackson Immunoresearch Laboratories. GFP expression allows the accurate identification and isolation of FoxP3+ Tregs. Congenic Thy1.1 mice (Cby.PL(B6)-Thyla/ScrJ) were obtained from Jackson Immunoresearch Laboratories. These animals carry the T lymphocyte-specific Thy1.1 allele. Donor T cells from Thy1.2 mice can be disctinguished from recipient Thy1.1 mouse T cells using anti=Thy1.2 antibodies. Mice were used at the age of 6-8 weeks.

Preparation of allogeneic, activated emTh-1 cells - The emTh-1 cells were generated and activated *in vitro* as described in Har-Noy et al, Leukemia Research (2009) 33:525-538 and Har-Noy et al., Leukemia Research (2008) 32:1903-1913. Spleen cells from C57BL/6 mice were harvested and treated with ammonium chloride-potassium (ACK) buffer for lysis of red blood cells. CD4⁺ T cells were then isolated using CD4⁺ microbeads and an autoMACS^{™} separator device (Miltenyi Biotec, Auburn, CA). Positively and negatively selected cells were routinely analyzed by flow cytometry to assess the purity of each fraction. These CD4⁺ T lymphocytes were expanded in RPMI medium (Gibco/BRL, Gaithersburg, MD) supplemented with 10% heat-inactivated fetal bovine serum (Gemini Bio-products, Woodland, CA), with anti-CD3 and anti-CD28-coated paramagnetic beads (CD3/CD28 T-cell Expander beads, Dynal/Invitrogen) at an initial bead:CD4⁺ cell ratio of 3:1, and in presence of 20 IU/mL recombinant mouse (rm)IL-2, 20 ng/mL rmIL-7, 10 ng/mL rmIL-12 (Peprotech, New Jersey) and 10 µg/mL anti-mouse IL-4 mAb (Becton Dickenson). Additional complete media containing rmIL-2, rmIL-7, anti-IL-4 mAb and CD3/CD28 beads was added to the culture daily from days 3 to 6 to maintain constant cell density (0.5-1 × 10⁶ cells/mL). The amount of beads added was calculated to maintain a 1:1 bead:cell ratio as the cells expanded. After 6 days in culture, the expansion of CD4⁺ T cells was approximately 60 to 100-fold the initial number of plated cells at day 0. Cells were harvested on day 6 and de-beaded by physical disruption and passage over a magnet. These cells were either used fresh or stored in liquid nitrogen for future use. A similar protocol was followed to generate emTh-1 cells from C57BL/6 mice.

Human emTh-1 cells were generated from healthy donor peripheral blood lymphocytes isolated by density centrifugation with lymphocyte separation medium (1.077; Eurobio). CD4 T cells were then isolated using human CD4 microbeads (Miltenyi Biotec) and cultured with human T-cell expander beads (Dynabeads; Invitrogen) in the presence of 20 IU/mL recombinant human IL-2 (rhIL-2), 20 ng/mL rhIL-7, 10 ng/mL rhIL-12 (Peprotech), and 10 g/mL anti-human IL-4 mAb (BD Bio- sciences). Human CD4 T-cell cultures were maintained as described in the previous paragraph for mouse cells. Human studies were approved by the institutional review board (IRB00005448; FWA00004218), with informed consent in compliance with the Declaration of Helsinki

Magnetic cell sorting-Spleens isolated from BALB/c or C57BL6 mice were harvested and dissociated. CD4⁺CD62L⁺, CD4⁺CD25⁺ and CD4⁺CD25⁻ T lymphocytes were purified by magnetic cell sorting using mouse CD4⁺CD62L⁺ naive T cell or CD4⁺CD25⁺ T regulatory cell isolation kits and an autoMACS^{™} separator according to the manufacturers' instructions (Miltenyi Biotec, Auburn, CA). The CD4⁺CD25⁺ T lymphocytes isolated by this technique express high level of the transcription factor FoxP3 and are endowed with immunosuppressive properties.

Conversion of CD4⁺CD62L⁺ T cells into CD4⁺CD25⁺FoxP3⁺ Treg--CD4⁺CD25 CD62L⁺ naive T cells were isolated from Balb/c mouse splenocytes as described above, were cultured in complete medium in 96-well plate (100 cells per well) and were activated with anti-CD3/anti-CD28 T cell expander beads (Dynabeads, Invitrogen) at a T lymphocyte:bead ratio of 1:1) in presence of TGF-β (5 ng/ml) for 72 hrs at 37°C. Some wells were treated with the supernatant of allogeneic activated CD4⁺ emTh-1 cells. Percentage of CD4⁺CD25⁺FoxP3⁺ and CD4⁺CD25⁺FoxP3⁻ cells were determined by flow cytometry analysis.

Flow cytometry analysis and antibodies--Cells (~10⁶) were washed in PBS containing 3% heat-inactivated fetal bovine serum and 0.09% sodium azide (Sigma Chemical) and were first incubated with an Fc receptor-blocking Ab (BD Biosciences Pharmingen, San Diego, CA) for 5 min, then with saturating amounts of the appropriate combination of fluorochrome-conjugated Ab for 40 min. Cells were then washed and analysed using a FACS calibur (Becton Dickinson Immunocytometry Systems, San Jose, CA). A minimum of 10,000 events were collected for each sample, and data analysis was performed with the CellQwest Pro software (Becton Dickinson Immunocytometry Systems). For FoxP3 detection, CD4⁺CD25⁺ or CD4⁺CD25⁻ T cells purified by magnetic cell sorting or converted CD4⁺CD25⁺ Treg generated *in vitro* were fixed, permeabilized, stained using an Allophycocyanin (APC) anti-mouse FoxP3 staining set following the provider's instructions (Clone FJK-16, eBioscience, San Diego, CA), and analyzed by flow cytometry. For the monitoring of CD4⁺CD25⁺ Treg, cells were first stained with fluorescein isothiocyanate (FITC)-conjugated anti-CD4 (rat IgG2b; BD Biosciences Pharmingen) and phycoerythrin (PE)-conjugated anti-CD25 (rat IgG1; BD Biosciences Pharmingen) antibodies. Then, cells were stained using eBioscience FoxP3 staining set as described above. The expression of the transcription factors Tbet and Gata-3 (expressed by Th-1 and Th-2 cells, respectively) were evaluated by intracellular staining using anti-mouse Tbet-phycoerythrin and anti-mouse Gata-3-phycoerythrin monoclonal antibodies (BD Biosciences Pharmingen). Isotype control antibodies were purchased from BD Biosciences Pharmingen (PE-conjugated rat IgG1, FITC-conjugated rat IgG2a) or eBioscience (APC-conjugated rat IgG1).

T cell proliferation and suppression assays--CD4⁺CD25⁺ and CD4⁺CD25⁻ T cells were purified from splenocytes and lymph node cells using Miltenyi isolation kits. The cells were cultured for 48 hrs in 96-well plates at 37°C either in complete medium or with activated emTh-1 cell supernatant and activated with plate-bound anti-CD3, soluble anti-CD28 and IL-2. In other experiments, untreated, activated emTh-1 cell supernatant pretreated or freshly isolated CD4⁺CD25⁻ T cells (1 × 10⁵) were co-cultured for 48 hrs in round-bottom 96-well plates with treated or untreated CD4⁺CD25⁺ T cells (1 × 10⁵). Anti-CD3/CD28 T cell expander beads - Dynabeads were added in all co-cultures (cell/bead=1/1). Bromodeoxyuridine (BrdU) (Millipore Corporation of Massachusetts) was then added for an additional 12 hrs. The cells were then fixed and the incorporation of BrdU detected by enzyme-linked immunosorbent assay (ELISA) according to the manufacturers' procedures (Millipore Corporation of Massachusetts). Cultures were set up in sixplicates.

Similar experiments were performed using human peripheral blood lymphocytes isolated by density centrifugation with 1.077 lymphocyte separation medium. CD4⁺CD25⁺ and CD4⁺CD25⁻ T cells were purified from total peripheral blood mononuclear cells using a human regulatory T-cell isolation kit (Miltenyi Biotec). Cells were cultured for 24 hours in 96-well plates at 37°C either in complete medium or with human emTh-1 supernatant, and activated with plate-bound anti-CD3 (5 ng/mL), soluble anti-CD28 (5 ng/mL), and IL-2 (20 IU/mL). CD4⁺ CD25⁻ responder T cells were then stained using the Cell Trace Violet cell-proliferation kit according to the manufacturer's procedure (Invitrogen). Labeled cells were cocultured with CD4⁺ CD25⁺ T cells (1x10⁵) with human anti-CD3/CD28 T-cell expander beads (cell:bead ratio 1:1) and cell division was analyzed by flow cytometry after 72 hours, as indicated by the manufacturer.

Detection of cytokine production by ELISA--The concentrations of IFN-γ and TNF-α in cell culture supernatants were determined using enzyme-linked immunosorbent assay (ELISA) kits according to the manufacturers' procedures (eBiosciences).

12B1 leukemia cells and tumor generation--The murine leukemia cell line 12B1 was obtained by retroviral transformation of BALB/c bone marrow cells with the human *bcr*-*abl* (b₃a₂) fusion gene. These cells express the p210 bcr-abl protein. This is an aggressive leukemia, with the 100% lethal dose (LD₁₀₀) being 100 cells after tail vein injection. The cells were cultured (37°C, 5% CO₂) in RPMI medium (Gibco/BRL, Gaithersburg, MD) supplemented with 10% heat-inactivated fetal bovine serum (Gemini Bio-products, Woodland, CA). 12B1 cells were obtained from Dr Wei Chen (Cleveland Clinic, Cleveland, OH), were tested routinely and found to be free of *Mycoplasma* contamination.

For tumor generation, 12B1 cells were first washed 3 times in PBS (Gibco/BRL), then counted and adjusted to a concentration of 5 × 10⁴ cells/mL. Female BALB/c mice were injected with 0.1 mL (5 × 10³ cells) subcutaneously in the right groin and were monitored for tumor development.

CRCL preparation--12B1 tumor-derived CRCL were prepared. Briefly, tumor tissues were homogenized in detergent-containing buffers and the obtained high-speed supernatants were subjected to FS-IEF in a Rotofor device (Bio-Rad) at 15W constant power. Fractions were harvested and analyzed for chaperone protein content. Fractions of interest were pooled and prepared as vaccines by dialysis, detergent removal, and centrifugal concentration. The endotoxin level contained in the CRCL preparation was determined using the Limulus Amebocyte Lysate (LAL) assay kit (Cambrex Bio Science, Walkersville, MD) according to the manufacturer's instructions. The level of endotoxin in CRCL was lower than that in media control (<0.01 EU/µg). 12B1 CRCL was used for *in vivo* vaccination of mice.

Tumor growth *in vivo* and combination immunotherapy--BALB/c mice were injected with 5 × 10³ viable 12B1 cells in the right groin on day 0. Allogeneic (C57BL6) activated CD4⁺ cells (10⁵ cells/mouse) or 12B1-derived CRCL vaccine (25 µg/mouse) or cells plus CRCL were administered on days +3, +7 and +14. The treatments were given in the right footpads in a total volume of 100 µl. Tumor growth was monitored every other day and mice were euthanized when tumor volume reached 4000 mm³. Tumor-free survival was compared between the different treatment groups.

Depletion of immune cells *in* vivo--Mice were depleted of NK cells by intraperitoneal injections with anti-asialo GM1 antibodies (25 µl/mouse, 1/8 diluted with PBS; Wako Chemicals, Osaka, Japan) on days -1, +3 and +5.

Statistics--Kaplan-Meier curves were generated and analyzed by log-rank statistics to determine survival percentages and differences between the treatment groups. In other experiments, Student's t tests were used to determine significant differences (p<XXXX) between groups.

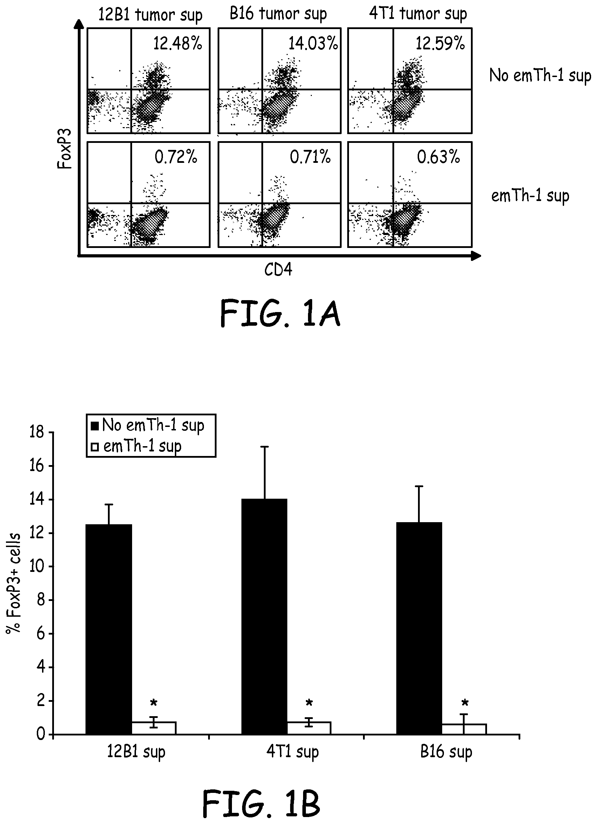

CD4⁺CD25⁻CD62L⁺ naive T cells were isolated from mouse spleens using magenetic activated cell sorting. The cells were activated using T-cell expander beads (cell:bead ratio 1:1), resuspended in the culture medium from different tumor cell lines (12B1, B16, 4T1) and treated with the supernatants of emTh-1 cells. In Fig. 1A, treated cells were incubated in a 96-well plate at 37°C for 72hrs and FoxP3 expression was determined using Flow Cytometry analysis. Fig. 1B shows the pooled data from three independent experiments. The data was graphed and Student's t tests were used to analyze the diagrams.

As shown in Figs. 1A and 1B, Th-1 cell supernatants impaired the differentiation of CD4⁺CD25⁺FoxP3⁺ Treg from naive CD4⁺CD25⁻FoxP3⁻ T cells induced by tumor cells or TGF-β *in vitro.* Treg expansion may result from the conversion of CD4⁺CD25⁻FoxP3⁻ T cells into CD4⁺CD25⁺FoxP3⁺ or from the proliferation of naturally occurring Treg. The transcription factor FoxP3 is required for the induction of Treg suppressive function, and its expression in non-regulatory cells converts them into immunosuppressive cells. The role of soluble factors produced by effector-memory Th-1 cells was examined. In particular, studies were done to see if TGF-β may negatively regulate the production of Treg from naive T cells induced by different TGF-β-secreting tumors *in vivo.* 12B1, B16 melanoma and 4T1 cells are known to be capable of secreting TGF-β in culture. The culture of naive CD4⁺CD62L⁺ T cells with either of these 3 tumor cell lines triggered their differentiation into FoxP3⁺ T cells (Figure 1A). Tumor-induced FoxP3 expression was however significantly dampened by the presence of the supernatant of activated emTh-1 cells during the culture (Fig. 1A,B).

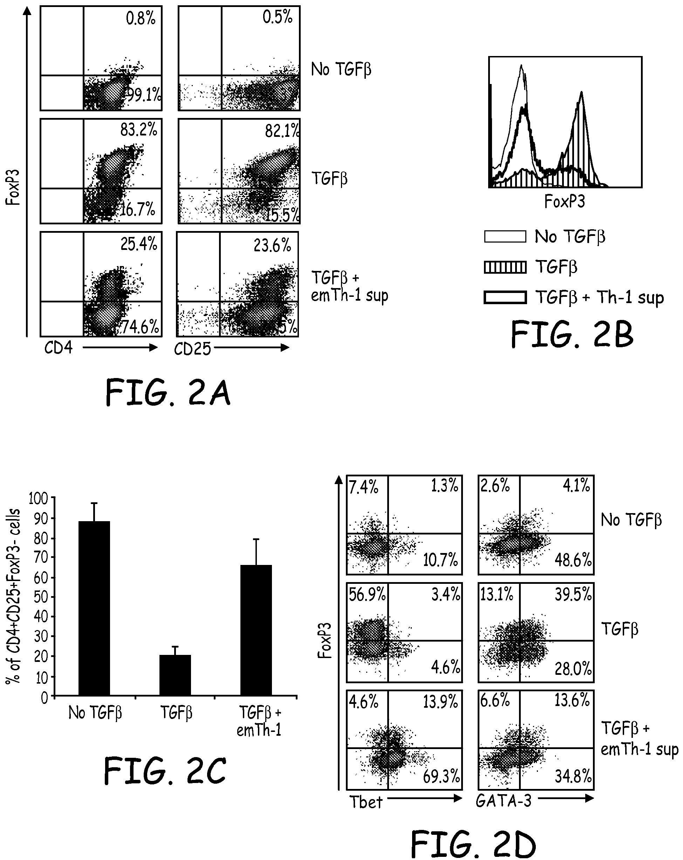

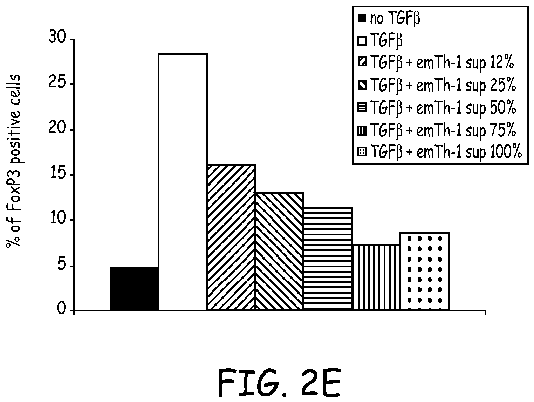

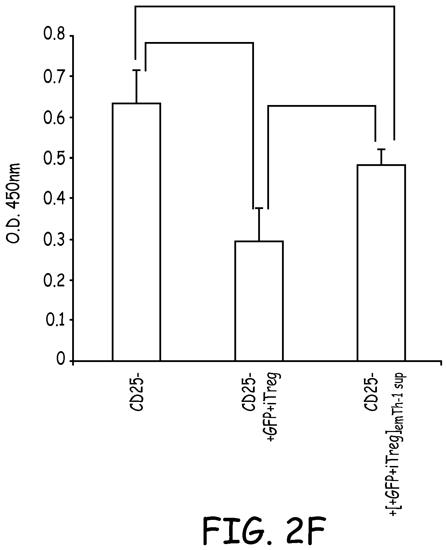

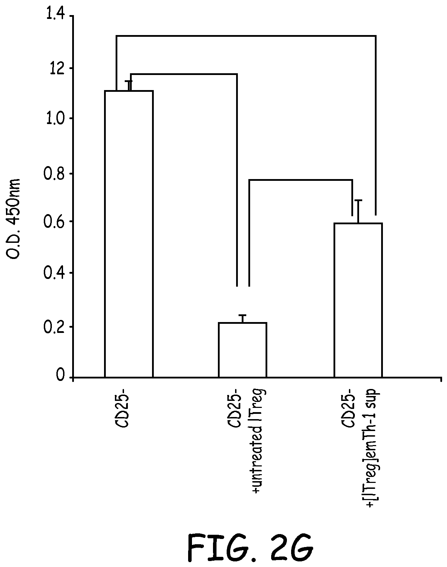

CD4⁺CD25⁻CD62L⁺ naive T cells were cultured for 72 hours with T-cell expander beads (cell:bead ratio 1:1) with or without TGF-β (5ng/mL) in the presence or absence of the emTh-1 supernatant (emTh-1 sup). Cells were then analyzed by flow cytometry. Fig. 2A and Fig. 2B ae representative dot plots or histogram plots from 10 independent experiments. Fig. C is a percentage of CD4⁺CD25⁺FoxP3⁻ activated T cells in total CD4+ T lymphocytes. P<.01, a significant difference compared with cells cutltured without emTh-1 supernatant. Fig. 2D is the expression of the transcription factors FoxP3, Tbet and GATA-3 was determined in CD4⁺CD25⁻ CD62L⁺ T cells cultured for 72 hours with T-cell expander beads with or without TGF-β1 treated or not with emTh-1 sup. Representative results of three experiments are shown in Fig. 2D. The data in Fig. 2A-2G indicate that emTh-1 supernatants significantly inhibited TGF-β-induced conversion of naive T cells into FoxP3⁺ T lymphocytes (Figs. 2A and 2B). Furthermore, the number of activated effector (CD25⁺FoxP3⁻) cells were augmented by the allogeneic emTh-1 supernatant (Fig. 2C) The effect was seen in a dose dependent manner (Fig. 2E). TGF-β-induced conversion of naive CD4⁺CD25⁻FoxP3⁻T cells isolated from FoxP3EGFP-transgenic mice was performed in the presence or absence of emTh-1 supernatant. GFP-positive (ie, FoxP3-expressing) cells were then sorted and their suppressive activity evaluated. The results demonstrated that the function of these residual FoxP3-expressing iTregs was impaired (Fig. 2F). In addition, emTh-1 supernatant added to FoxP3⁺ iTregs that had been previously converted significantly impaired their immunosuppressive function (Fig. 2G).

Effector-memory Th-1 supernatants were evaluated to see if they may skew TGF-(3-induced Foxp3⁺ T cell differentiation toward pro-inflammatory cell lineage. The transcription factors T-bet and GATA-3 are predominantly expressed by Th1 or Th2 cells, respectively. Flow cytometry analysis indicated that the majority of CD4⁺ T cells obtained after 72 hrs of culture in presence of TGF-β plus Th-1 cell supernatants expressed low level of Gata-3 and FoxP3. Most of the obtained cells displayed a Tbet-positive phenotype consistent with a Th-1 polarization (Fig 2D). These results thus indicated that the emTh-1 cells produce soluble factors capable of switching TGF-P-dependent polarization of naive T cells from FoxP3⁺ Treg to the pro-inflammatory Th-1 lineage.

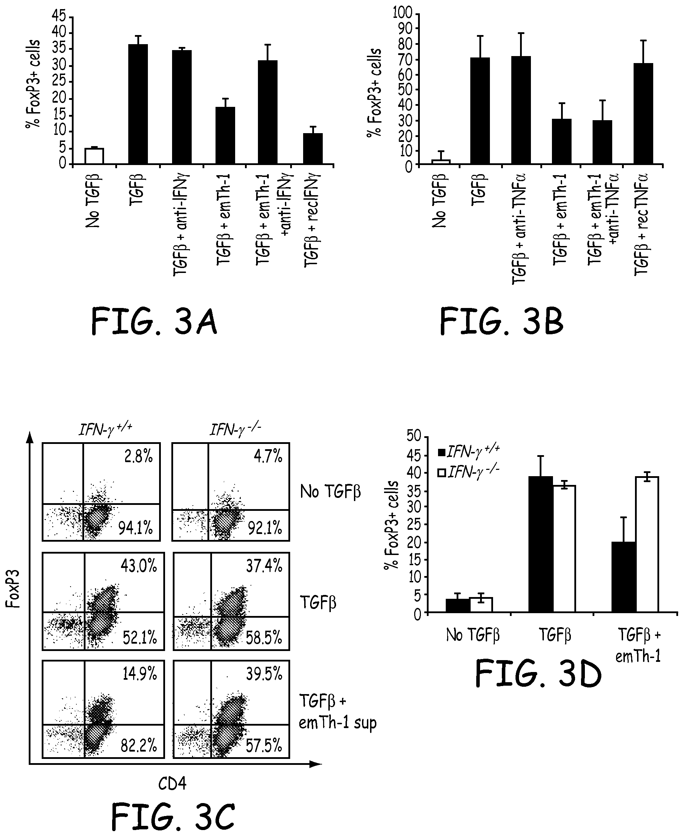

CD4⁺CD25⁻CD62L⁺ naive T cells were cultured for 72 hours with T-cell expander beads with or without TGF-β1 in the presence or absence of emTh-1 supernatant with or without blocking antibodies against (Fig. 3A) mouse IFN-γ, or (Fig. 3B) mouse TNF-α. In Figs. 3C and 3D, CD4⁺CD25⁻CD62L⁺ naive T cells were isolated from IFN-γR^{-/-} mice spleens and cultured for 72 hours with T-cell expander beads with or without TGF-β1 in the presence or absence of emTh-1 supernatant treated with TGF-β1 and in the presence or absence of emTh-1 supernatant.. Percentages of the FoxP3 expressing cells were determined by Flow Cytometer.

The involvement of the major cytokines produced by Th-1 cells was examined. High levels of IFN-γ and TNF-α were detected in the supernatants from Th-1 cell culture. Neutralization, using blocking antibodies, of IFN-γ but not TNF-α abrogated the effects of emTh-1 supernatant, restoring TGF-β induced conversion of naive cells to FoxP3-expressing T cells (Figs. 3A and 3B). This data indicated that recombinant IFN-γ, but not TNF-α, impaired the negative modulation of TGF-β induced generation of FoxP3⁺ T cells (Figs. 3A and 3B). These results were confirmed using IFN-γR^{-/-} mice. The data depicted in Figs. 3C and 3D indicated that the conversion of CD4⁺ naive T cells isolated from IFN-γR^{-/-} mice into FoxP3⁺ T cells was not modified by Th-1 cell supernatants (Figs.3C and 3D). IFN-γ therefore represented a major mechanism responsible for Th-1-mediated inhibition of FoxP3⁺ T lymphocytes generation.

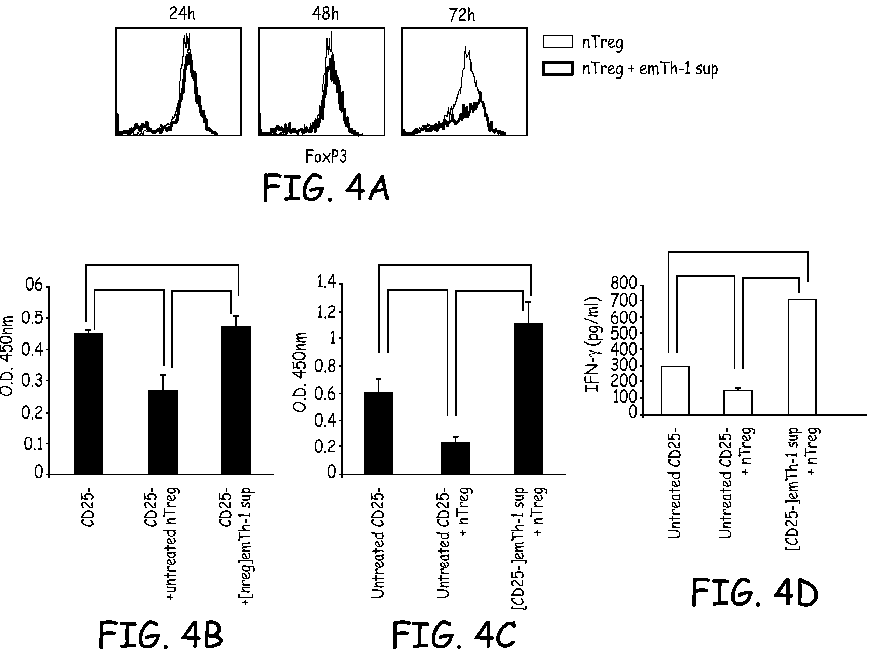

In Fig. 4A, CD4⁺CD25⁺ nTregs were isolated from BALB/c mouse lymphoid tissues and cultured for the indicated periods of time with plate-bound anti-CD3 (5 ng/mL), soluble anti-CD28 (5 ng/mL), and IL-2 (20 IU/mL) with or without emTh-1 supernatant. FoxP3 expression was then determined using flow cytometry. In Fig. 4B, CD4⁺CD25⁺ nTregs were cultured for 48 hours with plate-bound anti-CD3, soluble anti-CD28, and IL-2 with or without emTh-1 supernatant. Cells were then washed extensively with complete medium. Responder CD4⁺ CD25⁻T lymphocytes were stimulated with anti-CD3/anti-CD28 T-cell expander beads in the absence (CD25⁻) or presence of untreated nTregs (CD25⁻ + untreated nTreg) or in the presence of emTh-1 supernatant-treated nTregs (CD25⁻ [nTreg]_{emTh-1sup}). Responder CD4⁺CD25⁻ T-lymphocyte proliferation was determined after 48 hours using BrdU incorporation assays. NS, nonsignificant; P <.001, a significant difference compared with responder CD25⁻ T cells cultured with untreated Tregs. In Fig. 4C, CD4⁺CD25⁻ T lymphocytes were first treated ([CD25⁻]_{emTh-1sup}) or not (untreated CD25⁻ ) for 48 hours with emTh-1 supernatant, washed extensively with complete medium, and cocultured for 48 hours with freshly isolated CD4⁺CD25⁺ nTregs (+nTreg). Proliferation of responder CD25⁻ T cells was then determined using BrdU incorporation assays. P < .001. In Fig. 4D, IFN- γ concentration was assessed in the cocultures as described for Fig. 4C.

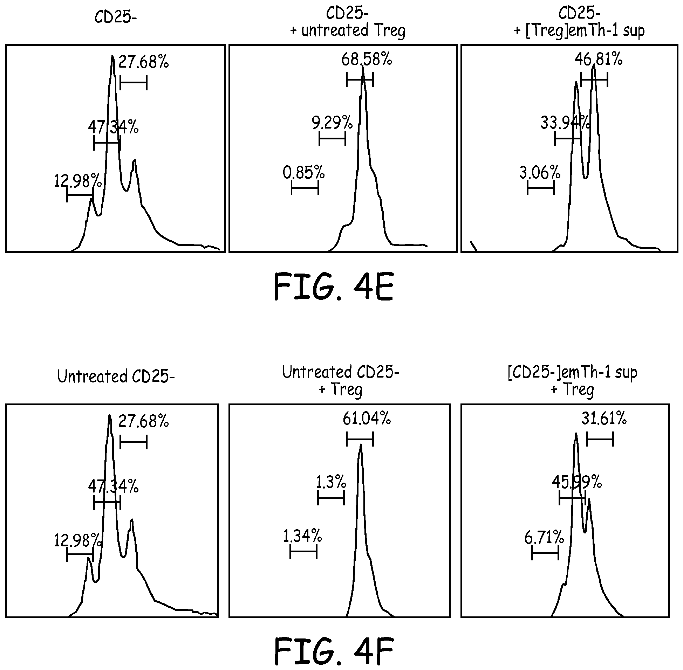

In Fig. 4E, CD4⁺CD25⁺ Treg were isolated from human PBMC and were exposed to human emTh-1 cell supernatant for 24 hours. Cell Trace Violet-labeled CD25⁻ responder T cells were then added to untreated Treg (CD25 ⁻ + untreated Treg) or emTh-1 pretreated Treg (CD25⁻ + [Treg]^{emTh-1sup}). In Fig. 4F, Human CD4⁺CD25⁻ T lymphocytes were first treated ([CD25⁻]^{emTh-1sup}) or not (untreated CD25⁻) with emTh-1 supernatant, then labeled with Cell Trace Violet and cocultured with freshly isolated CD4⁺CD25⁺ Treg (+Treg). Responder CD 25⁻ T-cell proliferation was determined using flow cytometry analysis as described in material and methods.

The results in Fig. 4A demonstrated that FoxP3 expression in nTregs was not impaired by the emTh-1 supernatant. nTregs isolated from mouse lymphoid tissues were activated with anti-CD3 and anti- CD28 antibodies and IL-2. The cells were cultured in either complete medium or emTh-1 supernatant for 48 hours and then washed before being cocultured with freshly isolated CD4⁺CD25⁻cells for an additional 48 hours. The ability of emTh-1 supernatant treated or untreated nTregs to inhibit the proliferation of CD4⁺CD25⁻ cells was analyzed using BrdU incorporation assays. The data indicated that emTh-1 supernatant (Fig. 4B) or IFN-gamma significantly inhibited the capacity of Tregs to suppress CD4⁺CD25⁺ conventional T-cell proliferation. Similar results were obtained using human CD4⁺CD25⁺ Tregs and CD4⁺CD25⁻ responder T cells isolated from peripheral blood lymphocytes (Fig. 4E).

T cells were incubated for 48 hours with emTh-1 supernatant and then cocultured with freshly isolated CD4⁺CD25⁺FoxP3⁺ nTregs. Neither the proliferation (Fig. 4C) nor the production of IFN-gamma (Fig. 4D) of CD4⁺CD25⁻ T cells pretreated with emTh-1 supernatant was suppressed by Tregs. Similar results were obtained when human CD4⁺CD25⁺ responder T cells pre-incubated with emTh-1 supernatant were exposed to human CD4⁺CD25⁺ Tregs (Fig. 4F). These data indicated that emTh-1 cells not only impair the inhibitory function of Tregs, but also induce resistance of effector T cells to Treg-mediated inhibition.

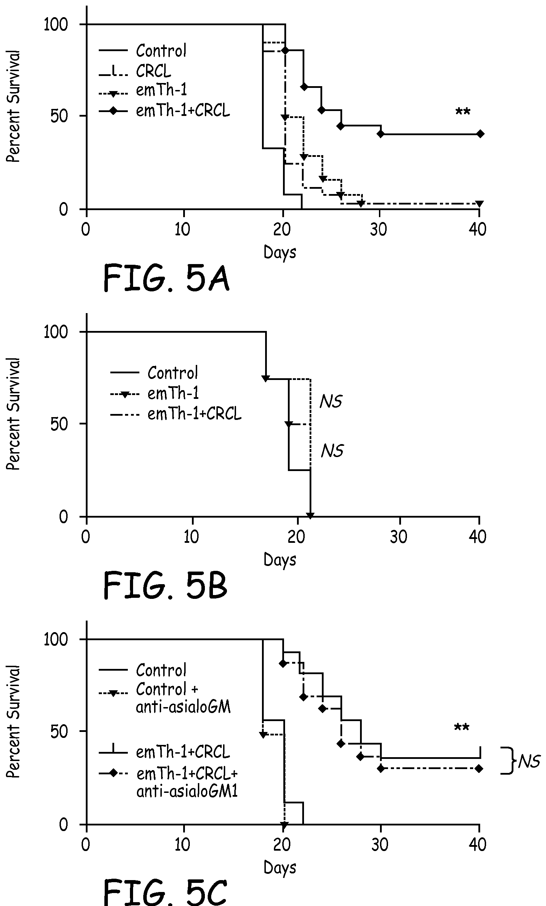

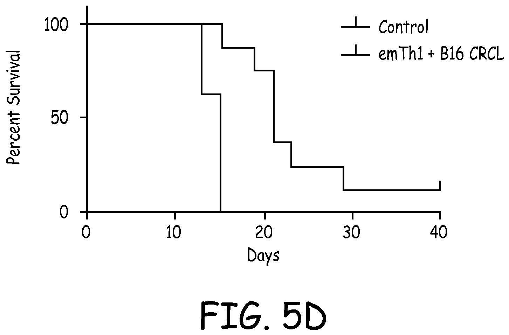

Naive Balb/c mice were injected with 5 × 10³ viable 12B1 cells in the right groin on day 0. In Fig. 5A, vaccinations were carried out on days +3, +7 and +14. The treatments were given in the right footpads as described in "Material and methods". Survival of mice was monitored and displayed in the Kaplan-Meyer plots. In Fig. 5B, SCID mice were injected with 5 × 10³ viable 12B1 cells in the right groin on day 0 and treated with either PBS, activated emTh-1 or the combination of activated emTh-1 +CRCL by the footpad injections on days +3, +7 and +14. In Fig. 5C, immunocompetent Balb/c mice were injected with tumor cells and treated with either PBS or the combination of activated emTh-1 + CRCL on days +3, +7 and +14. In some groups of mice, NK cells were depleted using i.p. injections of anti-asialo GM1 antibodies on days -1, +3 and +5 as described "Material and methods". In all the experiments survival of mice was monitored and displayed in the Kaplan-Meyer plots. In Fig. 5D, C57BL/6 mice were injected with 5 × 10⁵ B16 cells on day 0. Animals were then treated (day 3, 5 and 7) wit B16 derived CRCL plus emTh-1 + B16 CRCL). Tumor volume was monitored every other day as described in material and methods. Animal survival is depicted using the Kaplan-Meyer analysis.

To induce tumor specific immunity, allogeneic effector-memory Th-1 cell-based immunotherapy was evaluated to see if it can be combined with a personalized vaccine as CRCL derived from the same type of tumor. As shown in the results of Fig. 5, allogeneic emTh-1 cells can be safely delivered in vivo and efficiently combined with tumor-derived chaperone-rich cell lysate (CRCL) to treat mice with 12B1 leukemia. Established 12B1 tumors in naive Balb/c mice were used and confirmed that allogeneic effector-memory Th-1 cell-based immunotherapy can be efficiently combined with CRCL vaccination strategy resulting in a significant tumor-free survival of treated animals (Fig. 5A). Similar results were obtained with a B16 melanoma model that consisted of B16 tumor-bearing C57BL/6 mice treated with B 16-derived CRCL plus emTh-1 cells generated from Balb/c mice (See Fig. 5D).

The role of T cells in the anti-tumor responses induced by CRCL plus activated emTh-1 was defined by utilizing SCID and NUDE mice. 12B1 tumor-bearing SCID mice were treated with CRCL plus activated emTh-1 3, 7 and 14 days after tumor cell inoculation as described above. CRCL plus activated emTh-1 immunotherapy did not improve survival of 12B1 tumor-bearing SCID mice (Fig. 5B). Similar results were obtained using NUDE mice. These data indicated that the protective effects of allogeneic Th-1 plus CRCL combination immunotherapy against 12B1tumors depend on a T cell-mediated immune response. Consistent with these data, the *in vivo* depletion of NK cells in immunocompetent mice using anti-asialo-GM1 antibodies did not significantly impair the therapeutic efficacy of allogeneic Th-1 plus CRCL treatment, indicating that NK cells do not play a major role in the antitumor immune responses induced by the combination therapy (Fig. 5C). Thus, the tumor-protecting effects of immunotherapy based on allogeneic activated emTh-1 cells in combination with CRCL vaccine depend on T lymphocytes.

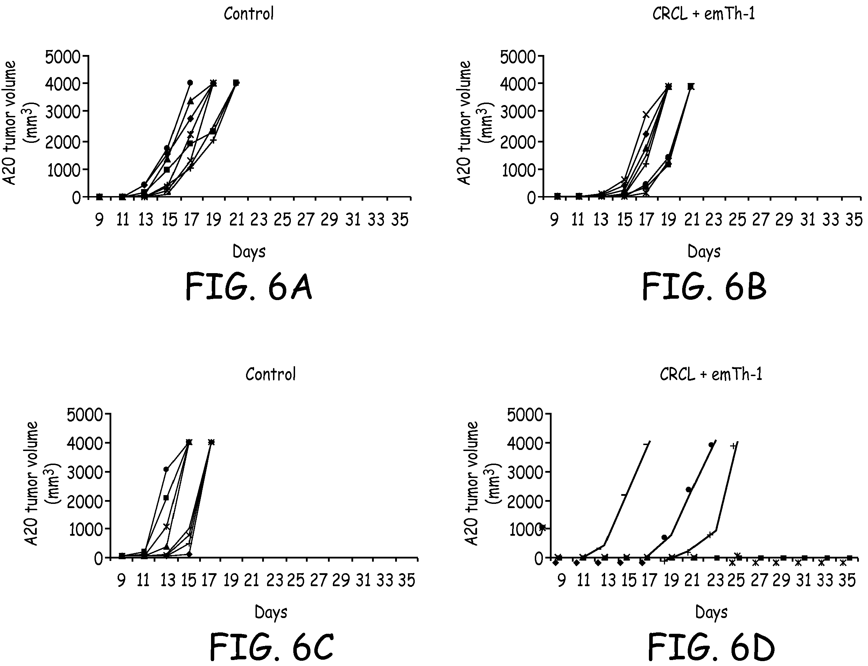

Naive BALB/c mice were injected with 5×10³ 12B1 cells (subcutaneously in the right groin) on day 0 and vaccinated as described for Fig. 5. Surviving tumor-free mice were then rechallenged with 5×10³ 12B1 cells given subcutaneously in the right groin and 1×10⁶ A20 cells given subcutaneously in the left groin on day 45. Tumor volume was determined every other day. In Fig. 6A, A20 tumor volume monitored in control mice. In Fig. 6B, A20 tumor volume monitored in emTh-1 plus CRCL-treated mice. In Fig. 3C, 12B1 volume monitored in control group. In Fig. 3D, 12B1 tumor volume monitored in emTh-1 plus CRCL-treated animals. Results are representative of 2 independent experiments.

Surviving mice treated with CRCL plus allogeneic activated emTh-1 cells were rechallenged with the parental 12B1 tumor cells in the right groin and with an unrelated B cell leukemia (A20, H-2D) in the opposite groin. A20 tumors developed in all 8 mice in both treated and control groups (Figs. 6A and 6B). 5 out of 8 mice were protected against 12B1 tumor rechallenge in the CRCL plus allogeneic effector-memory Th-1 cell group and two showed the delay in tumor growth, while all control mice developed 12B1 tumors (Figs. 6C and 6D). These results thus indicate that combination immunotherapy consisting of CRCL plus allogeneic activated emTh-1 cells induces long lasting tumor-specific immunity in the majority of treated animals.

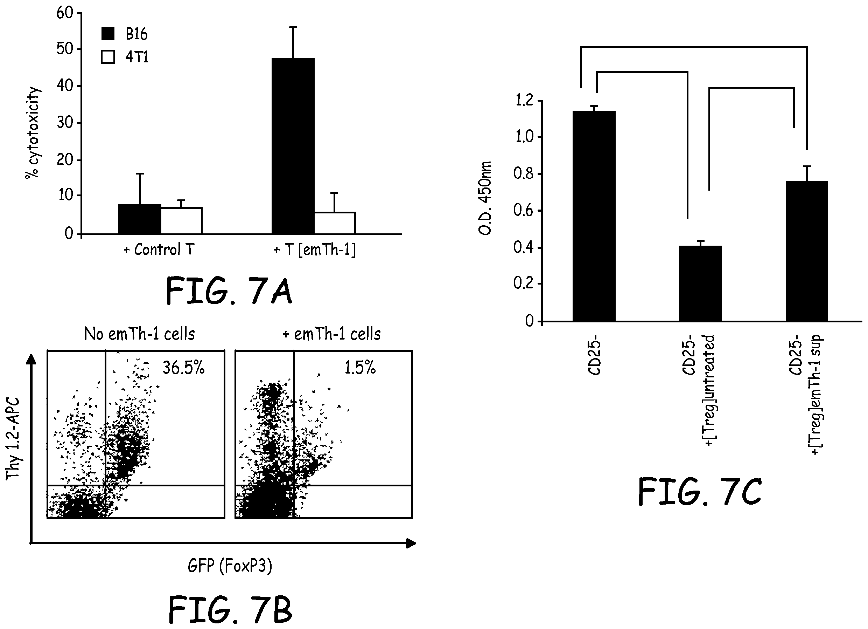

In Fig. 7A, emTh-1 plus CRCL immunotherapy inducee tumor-specific killer T lymphocytes. B16 tumor-bearing mice were injected with control PBS or were treated with B16-derived CRCL and allogeneic emTh-1 cells as indicated in materials and methods. Seven days after the last vaccination, splenocytes were harvested and incubated for 3 days with CRCL (25 micrograms/mL) and 50 U/mL IL-2. T lymphocytes were then purified on a nylon wool column and incubated for 36 hours with either B16 tumor cells or irrelevant 4T1 breast cancer cell targets as indicated (effector T cells to target tumor cells ratio 20:1). Cytotoxicity was determined. Control T, tumor cells cultured with T lymphocytes from mice injected with PBS; +T [emTh-1], tumor cells cultured with T lymphocytes from mice treated with CRCL plus emTh-1. In Fig. 7B, emTh-1 cells skewed the differentiation of CD4⁺CD25⁻ FoxP3⁻ naive T lymphocytes toward CD4⁺CD25⁺FoxP3⁻ effector T cells rather than Tregs in vivo. CD4⁺CD25⁻FoxP3⁻ naive T lymphocytes isolated from Thy1.2 FoxP3EGFP transgenic BALB/c mice (10⁷ cells) were transferred to 12B1 tumor-bearing congenic Thy1.1 BALB/c mice. Animals were treated with emTh-1 cells (+emTh-1 cells) or with control PBS (No emTh-1 cells). Endogenous T cells of recipient Thy1.1 mice express the Thy1.1 but not the Thy1.2 antigen, which allows the specific tracking and identification of Thy1.2 T lymphocytes in Thy1.1 mice. Spleens were harvested and dissociated, and conversion of transferred naive Thy1.2 CD4⁺CD25⁻FoxP3⁻ T cells into GFP+ (FoxP3-expressing) Treg in vivo was determined by evaluating the frequency of Thy1.2 GFP+ cells after gating on the CD4⁺ T-cell population using flow cytometry. In Fig. 7C, emTh-1 cells impaired Treg suppressive function in vivo. Responder CD4⁺CD25⁻ T lymphocytes were stimulated with anti-CD3/anti-CD28 T-cell expander beads in the absence (CD25⁻) or presence of Tregs isolated from the draining lymph nodes of untreated tumor-bearing mice (CD25⁻ + [Treg]untreated) or of tumor-bearing mice treated with emTh-1 (CD25⁻+[Treg]emTh-1). Responder CD4⁺CD25⁻ T-lymphocyte proliferation was determined after 48 hours using BrdU incorporation assays.

The antitumoral function of T cells isolated from CRCL plus emTh-1 cell-treated mice was analyzed. Lymphocytes purified from the spleens of animals receiving the combination therapy were capable of specifically killing parental tumor cells but not irrelevant target cancer cells (Fig. 7A). EmTh-1 cells were able to skew the differentiation of naive Thy1.2 CD4⁺CD25⁻FoxP3⁻ T lymphocytes transferred to 12B1 tumor-bearing congenic Thy1.1 mice toward Thy1.2 CD4⁺CD25⁺FoxP3⁻ effector T-cells rather than Thy1.2 CD4⁺CD25⁺FoxP3⁺ Tregs (Fig. 7B). In addition, the suppressive function of Tregs isolated from tumor-bearing animals treated with emTh-1 cells was significantly reduced (Fig. 7C). This confirmed that the effects of emTh-1 cells on Tregs observed in vitro also occur in vivo, and also demonstrated that the mechanism by which emTh-1 cells augment the efficacy of CRCL vaccination involves the inhibition of tumor-induced Tregs.

Although the present invention has been described with reference to preferred embodiments, workers skilled in the art will recognize that changes may be made in form and detail without departing from the scope of the invention.

## Claims

1. A therapeutic composition for use in treating a cancerous tumour in a patient, the composition comprising activated allogeneic emTh-1 cells and a chaperone rich cell lysate derived from the tumour, wherein the composition generates tumour-specific immunity in the patient such that the composition decreases the immunosuppressive activity of tumour-induced Treg cells in the patient while simultaneously promoting a therapeutic effect in the patient, and wherein, in use, the emTh-1 cells and the tumour-derived chaperone rich cell lysate are administered 3-6 times and at intervals of about 3-10 days by intradermal injections at the same location, followed then by an intravenous infusion of the emTh-1 cells.

2. The composition for use according to claim 1 wherein the Treg cells are CD4+CD25+FoxP3+.

3. The composition for use according to claim 1 wherein the suppressive activity of the Treg cells is inhibited by decreasing the conversion of naive T cells (CD4+CD25-FoxP3-) to iTreg cells.

4. The composition for use according to claim 1 wherein the suppressive activity of the Treg cells is by inhibition of nTreg cells.

5. The composition for use according to claim 1, wherein, in use, the activated allogeneic emTh-1 cells are administered prior to the tumour-derived chaperone rich cell lysate.

## Patentansprüche

1. Therapeutische Zusammensetzung zur Verwendung bei der Behandlung eines Krebstumors in einem Patienten, wobei die Zusammensetzung aktivierte allogene emTh-1-Zellen und ein aus dem Tumor stammendes chaperonreiches Zelllysat umfasst, wobei die Zusammensetzung eine tumorspezifische Immunität in dem Patienten erzeugt, so dass die Zusammensetzung die immunsuppressive Aktivität von tumorinduzierten Treg-Zellen in dem Patienten verringert und gleichzeitig eine therapeutische Wirkung in dem Patienten fördert, und wobei bei der Anwendung die emTh-1-Zellen und das vom Tumor stammende chaperonreiche Zelllysat 3-6 Mal und in Abständen von etwa 3-10 Tagen durch intradermale Injektionen an derselben Stelle verabreicht werden, gefolgt von einer intravenösen Infusion der emTh-1-Zellen.

2. Zusammensetzung zur Verwendung gemäß Anspruch 1, wobei die Treg-Zellen CD4+CD25+FoxP3+ sind.

3. Zusammensetzung zur Verwendung gemäß Anspruch 1, wobei die suppressive Aktivität der Treg-Zellen gehemmt wird, indem die Umwandlung von naiven T-Zellen (CD4+CD25-FoxP3-) in iTreg-Zellen verringert wird.

4. Zusammensetzung zur Verwendung gemäß Anspruch 1, wobei die suppressive Aktivität der Treg-Zellen durch Hemmung von nTreg-Zellen erfolgt.

5. Zusammensetzung zur Verwendung gemäß Anspruch 1, wobei bei der Anwendung die aktivierten allogenen emTh-1-Zellen vor dem vom Tumor stammenden chaperonreichen Zelllysat verabreicht werden.

## Revendications

1. Composition thérapeutique destinée à une utilisation dans le traitement d'une tumeur dans un patient, la composition comprenant des cellules emTh-1 allogéniques activées et un lysat cellulaire riche en protéine chaperonne issu de la tumeur, la composition générant une immunité spécifique de tumeur dans le patient de sorte que la composition diminue l'activité immunosuppressive de cellules Treg induites par la tumeur dans le patient tout en favorisant simultanément un effet thérapeutique dans le patient, et, en utilisation, les cellules emT-1 et le lysat cellulaire riche en protéine chaperonne sont administrées 3 à 6 fois et à des intervalles d'environ 3 à 10 jours par injections intradermiques au même endroit suivies ensuite d'une perfusion intraveineuse de cellules emTh-1.

2. Composition destinée à une utilisation suivant la revendication 1, dans laquelle les cellules Treg sont CD4+CD25+FoxP3+.

3. Composition destinée à une utilisation suivant la revendication 1, dans laquelle l'activité suppressive des cellules Treg est inhibée par diminution de la conversion de cellules T naïves (CF4+CD25-FoxP3-) en cellules iTreg.

4. Composition destinée à une utilisation suivant la revendication 1, dans laquelle l'activité suppressive des cellules Treg a lieu par inhibition de cellules nTreg.

5. Composition destinée à une utilisation suivant la revendication 1, dans laquelle les cellules emTh-1 allogéniques activées sont administrées préalablement au lysat cellulaire riche en protéine chaperonne issu de la tumeur.
